# EUROPEAN PATENT SPECIFICATION

(11) **EP 1 974 624 B1**
(45) Date of publication and mention of the grant of the patent: **12.09.2012**
(21) Application number: 06843789.6
(22) Date of filing: 28.12.2006
(51) Int. Cl.: A42B 3/16

(54) **DEVICE REDUCING WIND NOISE**
VORRICHTUNG ZUR VERRINGERUNG VON WINDGERÄUSCHEN
DISPOSITIF REDUISANT LE BRUIT DU VENT

(30) Priority: 30.12.2005 JP 2005381361
(43) Date of publication of application: 01.10.2008
(73) Proprietor: Mizuno, Ken, Inuyama-shi, Aichi 484-0868 (JP)
(72) Inventor: Mizuno, Ken, Inuyama-shi, Aichi 484-0868 (JP)
(74) Representative: Anohins, Vladimirs
(86) International application number: PCT/JP2006/326421
(87) International publication number: WO 2007/077983

(56) References cited:
- EP-A1- 1 329 168
- WO-A1-02/28212
- JP-A- 2001 020 122
- JP-U- 62 064 731
- JP-Y1- 43 004 723
- US-A- 5 323 493
- US-A- 5 477 564
- US-B1- 6 286 149

## Description

### TECHNICAL FIELD

The present invention relates to apparatuses using windshields to reduce the whistling sounds from wind audible to the riders of open-top vehicles.

### TECHNICAL BACKGROUND

In the following description, the directional terms "lateral", "vertical", and "front-to-rear" refer to the orientations of the apparatus according to the present invention as the apparatus is worn by the user of the apparatus with his head positioned upright such that the orientations for the rider correspond to the apparatus orientations. The term "outside" refers to locations farther from the body of the user than locations referred to as "inside". That is, as for the components attached in the vicinity of the left ear, the left side is positioned outside and the right side is positioned inside. The term "head" refers to the part of the body above the neck.

The phrase "surrounding sounds" refers to all the sounds audible to the user except for those produced in the vicinity of the ears.

When a person is riding an open-top vehicle with his ears exposed, he hears the whistling sounds of the apparent apparent headwind produced by the wind hitting his earlobes (4). This causes the following two undesirable conditions:
1. The sounds may hinder the rider from clearly hearing the surrounding sounds, thus increasing the danger in riding. In particular, the sounds greatly reduce the user's ability to notice vehicles approaching from behind.
2. The constant whistling sounds may place the rider under psychological stress and cause hearing difficulties.

If the ear lobes (4) of the rider are completely covered by the dome part of a helmet or a fabric or leather cover, none of the above-described whistling sounds will be generated. This, however, results in the problem of making it more difficult to hear the surrounding sounds. In addition, due to the poor heat dissipation in the vicinity of the user's ears, wearing such covers in summer becomes an agonizing experience.

Certain models of fabric or leather covers for completely covering the ear lobes (4) include holes provided in the vicinity of the ear holes (5) to permit sound propagation. One such example is described in claim 4 of Japanese Utility Model Application No. 8-11556. This solves the problem of difficulty in hearing the surrounding sounds. However, the poor heat dissipation in the vicinity of the user's ears still remains unsolved by this.

The following drawbacks are recognized in the apparatuses described in claims 1 and 3 of U.S. Patent No. 5,323,493: The windshields A (1) lessen the wind hitting the earlobes (4) to diminish the whistling sounds that would be heard if the apparatus was not used. However, the apparent headwind causes the windshields A (1) to vibrate, and this in turn creates a new inconvenience of generating high-frequency sounds audible to the user. In order to eliminate these sounds, the windshields A (1) need to be formed with high vibration damping properties. In reality, it is often the case that sufficiently high vibration damping properties are not realized, thus producing high-frequency sounds that are audible to the user. The windshields A (1) of the present invention correspond to the air deflectors of the above-referenced patent.

EP 1 3219 168 (WO 02/28212) discloses a windshield unit for reducing the whistling sounds from land, whereby a windshield and a sound insulating Plate form a single component, which may be filled to a helmet.

### DISCLOSURE OF THE INVENTION

### PROBLEMS TO BE SOLVED BY THE INVENTION

The apparatus according to the present invention is intended to offer the following four features:
1. To reduce the whistling sounds.
2. To allow the surrounding sounds to reach the ears of the user.
3. To provide sufficient heat dissipation in the vicinity of the ears of the user.
4. To prevent any additional sounds that the apparatus may produce from being audible to the user.

### MEANS TO SOLVE THE PROBLEMS

Windshields A (1) and sound insulating plates (2) are disposed such that the windshields and the insulating plates are separated from each other in a front-to-rear direction and secured to the helmet worn by the user forward of the user's ears. Each windshield A (1) provides cover forward of the user's earlobe (4) or from the front and extending to the side thereof, and the sound insulating plate (2) is positioned between the ear hole (5) and the windshield A (1). No cover is provided that covers the entire ear. Neither the windshield A (1) nor the sound insulating plate (2) comes into contact with the earlobe (4). The ears are mostly exposed to the open air such that the earlobes (4) of the user are visible from the behind.

If necessary, an additional windshield B (25) is disposed forward of each windshield A (1) below the helmet (3). The differences between the windshield A (1) and the windshield B (25) will be discussed in details later in the Best Modes to Carry Out the Invention and the Embodiment sections.

### EFFECT OF THE INVENTION

The apparent headwind is blocked by the windshields A (1) and does not reach the earlobes (4) and the ear holes (5) of the user. This eliminates the whistling sounds that may have been audible before use of the apparatus. Simultaneously, as an adverse effect, the head wind causes the windshields A (1) to vibrate and thus produce high frequency sounds.

The apparent headwind is blocked by the windshields A (1) and does not reach the sound insulating plates (2). Accordingly, the sound insulating plates (2) do not vibrate or make any sounds. The aforementioned sounds generated by the vibration of the windshields A (1) are blocked by the sound insulating plates (2) without reaching the ear holes (5) of the user.

Due to these effects, no sounds produced by the apparent headwind are audible to the user.

Since the user's earlobes (4) are mostly exposed to the open air, a sufficient level of heat dissipation in the vicinity of the earlobes is secured. For the same reason, the surrounding sounds reach the ear holes (5) of the user without attenuation.

In comparison with claims 1 and 3 of U.S. Patent No. 5,323,493, the windshields A (1) may not be allowed to vibrate, thus reducing their weight and simplifying their structure.

As a secondary effect, as the rear sides of the sound insulating plates (2) reflect sounds produced behind the user, thus improving the audibility of the sounds coming to the user from behind. As an additional secondary effect, the windshields A (1) and the sound insulating plates (2) function as shock absorbers that may protect the sides of the user's head in an accident.

The objectives and the effects of providing the windshields B (25) which not form part of the invention are as follows:

If the only windshields provided are the windshields A (1), the apparent headwind is abruptly redirected within a short distance, such that if the lateral length (18) of the windshield A (1) is short, the wind would flow around the rear end of the windshields (1) toward the respective ear holes (5), thereby readily creating turbulence. Accordingly, the lateral length (18) of the windshields A (1) would have to be long in order to reduce the noise level in the vicinity of the ear holes (5). Increasing the lateral length (18) would spoil the appearance and increases the air resistance of the apparatus. Moreover, wind would strike against the front edges of the windshields A (1) and produce sound in the vicinity of the ear holes (5). As this would require sound insulating plates (2) to have very high sound insulating properties, the plates would have to be thick and over-sized.

When the windshields B (25) are installed, the wind directions is changed gently, thus reducing the occurrence of the aforementioned turbulence even if the lateral length of the windshields A (1) is short.

Additionally, the head wind generates sounds as it strikes against the front edges of the windshields B (26). However, the sources of these sounds are farther from the ear holes (5) than if the windshields A (1) alone are provided. Accordingly, these sounds are less likely to reach the ear holes (5), allowing size reduction, simplification, or omission of the sound insulating plates (2).

In a situation where the user leans his head forward with the helmet (3) on, providing the windshields B (25) will prove even more effective for the reason below.

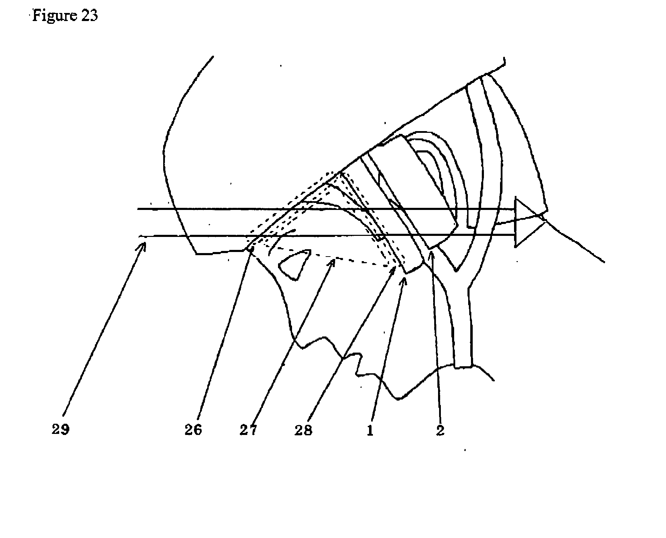

As shown in Figure 23, without the windshields B (25), the wind flowing through the surface of the dome portion of the helmet (3) turns at the lower end (26) of the helmet (3) and is directed around to the temples (27) of the user, eventually hitting the front edges (28) of the windshields A (1).

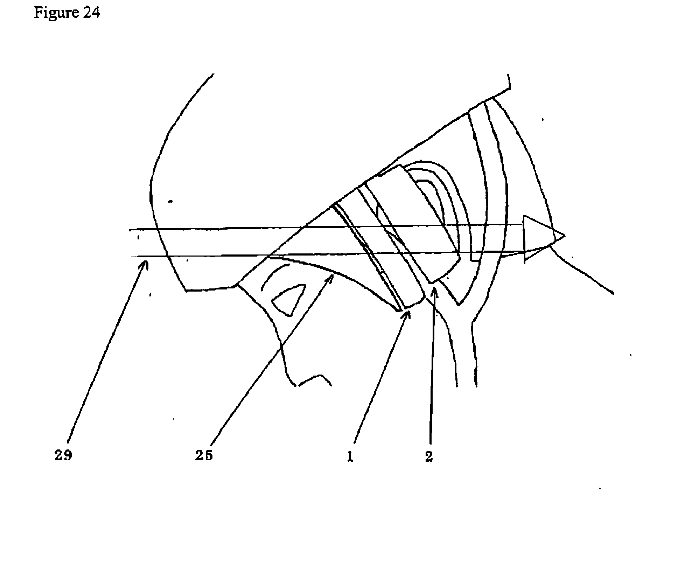

The domes of commercially available helmets have thicknesses of 2 to 3 centimeters and the majority of windshields A (1) have lateral lengths of about 3 centimeters. If the windshields B (25) fill the space between the lower end (26) of the helmet and the outermost protruding edges of the windshields B (25), as shown in Figure 24, the direction of the apparent headwind does not abruptly change in the vicinity of the ears, thus colliding neither the windshields A (1) nor the windshields B (25). That is, when the user leans his head forward wearing the helmet (3), the windshields B (25) fully eliminate the two causes of the whistling sounds from winds.

According to the present invention, it is assumed that the windshields B (25) are used in conjunction with the windshields A (1) and the sound insulating plates (2), it need not be mentioned that the windshields B (25) should be effective without one or both of the other two components.

One method of installing an apparatus to reduce the whistling sounds from winds on the temple (27) is disclosed in U.S. Patent No. 6,029,769. The effect of the invention of this U.S. patent is to employ a "shaped fiber filter" to change turbulence occurring in the vicinity of the apparatus into a laminar airflow and allow the airflow to blow past it. The windshields B (25) of the present invention are intended to allow the apparent headwind to flow several centimeters off the sides of the user's head. As such, the purposes of the two differ.

### BEST MODES FOR CARRYING OUT THE INVENTION

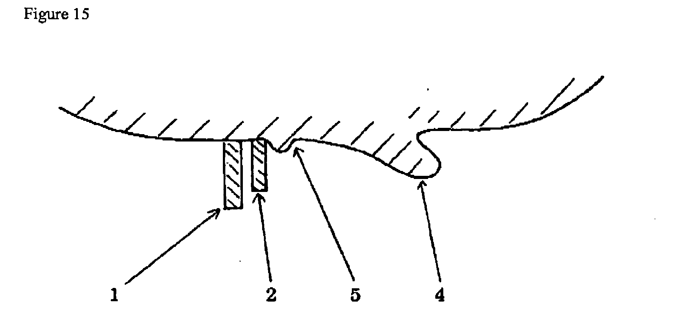

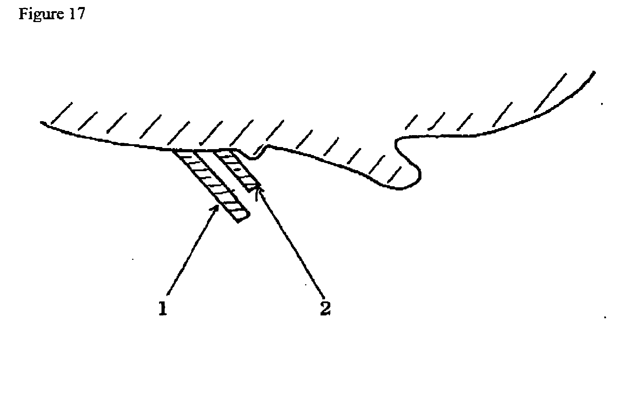

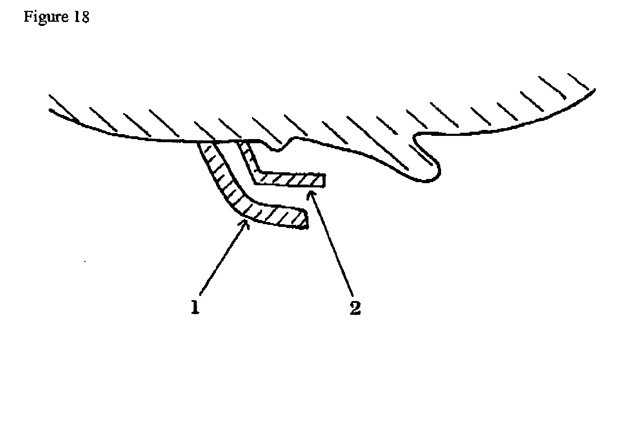

Methods for supporting the windshields A (1) and the sound insulating plates (2) will be described in embodiments later. The main points other than these methods are indicated in Items 1 to 14 below.
1. The windshields A (1) and the sound insulating plates (2) are in contact with the side surfaces of the user's head forward of the ear holes (5). The effect improves as these elements are brought closer to the ear hole (5).
2. The windshields A (1) and the sound insulating plates (2) should have basic shapes of flat, curved, or bent plates. They may not necessarily be formed as plates insofar as they do not cause excessive turbulence.
3. The windshields A (1) and the sound insulating plates (2) may have three possible orientations: they may be oriented straight outward, leaned rearward, and curved or bent at a halfway point. Figures15, 16, 17, and 18 show exemplary combinations of these orientations. These figures are cross-sectional views taken at the level of the ear holes (5). For the sake of simplicity, only one orientation is indicated in each embodiment below and the accompanying figure(s).
4. The windshields A (1) should have a vertical dimension of at least 3 centimeters. Although there is no upper limit to the vertical dimension, the apparatus does not increase its effectiveness if the windshields extend more than two centimeters beyond the upper or lower ends of the earlobes (4). As discussed below in Embodiments 1 and 2, when the windshields A (1) are supported on the helmet (3), the upper ends of the windshields A (1) are preferably connected to the helmet (3).
5. The windshields A (1) should have a minimum lateral dimension of 1 centimeter. If the windshields extend more than five centimeters beyond the upper or lower ends of the earlobes (4), the apparatus does not increase its effectiveness. If the upper and lower ends of the windshields A (1) are tapered or rounded, those portions may be less than one centimeter.
6. In the configurations shown in Figures 15 and 16, the rear ends of the windshields A (1) are positioned forward of the ear holes (5). In the configurations shown in Figures 17 and 18, there is no particular limitation on the rearward extension of the windshields A (1). However, if the rear ends are positioned rear of the center of the front-to-rear length of the earlobes (4), the apparatus does not significantly increase its effectiveness. Except for considerations regarding strong cross winds, there is no need to extend the windshields beyond the center points.
7. Preferably, the sound insulating plates (2) have sufficient dimensions to completely cover the space between the windshields A (1) and the ear holes (5). Preferably, the sound insulating plates (2) do not extend beyond the windshields A (1) as seen from the front. If the sound insulating plates (2) are allowed to extend beyond the windshields A (1), the amounts of extension at the upper and lower ends should be 5 mm or less as seen from the front.
8. The windshield A (1) and the sound insulating plate (2) should not come into contact with each other due to their vibration and deformation.
9. It is acceptable to leave gaps of about 5 mm between the user's head and the parts of the windshields A (1) and the sound insulating plates (2) that would otherwise come into contact with the user's head. Ideally, no gaps should be formed. For that purpose, a soft material or a cushioning material may be advantageously used for the elements that come into contact with the user's head. It may also be advantageous to employ a spring mechanism or the elasticity of the windshields A (1) and the sound insulating plates (2) to bring the windshields A (1) and the sound insulating plates (2) into intimate contact with the side surfaces of the user's head.
10. The materials of the windshields A (1) and the sound insulating plates (2) should be of such stiffness that they are not readily deformed by wind pressure or vibration. Rubbers and soft resins are the suitable materials. If these materials lack stiffness, a core material should be used to increase the stiffness. They should be securely fixed so as not to be easily displaced by the vibration and the wind pressure that occur during riding.
11. Holes (7) allowing passage of eyeglass temples may be formed through the windshields A (1) and the sound insulating plates (2) as required. It will be advantageous to plug the holes with sponges (8) to prevent wind from passing through. If the user does not wear glasses, the temple holes (7) may be plugged with separate elements.

This will be discussed further in Embodiment 3.
12. To accommodate a range of the physical constitutions of different users, it may be advantageous to provide a mechanism for adjusting the positions in which the windshields A (1) and the sound insulating plates (2) are attached.
13. In a a simplified apparatus, not forming part of the invention, each windshield A (1) and one sound insulating plate (2) may be made as a single, continuous element with the front portion (16) serving as the windshield and the rear portion (17) serving as the sound insulating plate. This corresponds to claim 2 of the present invention.
14. In another simplified apparatus, not forming part of the invention, the sound insulating plates (2) may be attached to the windshields A (1). In this case, the area of attachment between the two elements should be minimized rather than the entire opposing surfaces thereof being bonded. Alternatively, the sound insulating plates (2) may be attached to the windshields A (1) via support rods.

In the two simplified methods described above in Items 13 and 14, the sound insulating plates (2) no longer come into contact with the sides of the user's head, such that each sound insulating plate (2) does not completely cover the space between the windshield A (1) and the ear hole (5). In other words, this is an exception to what is discussed in Item 7.

Items 9, 10, 11, and 12 above are applicable to the windshields B (25) to be discussed below.

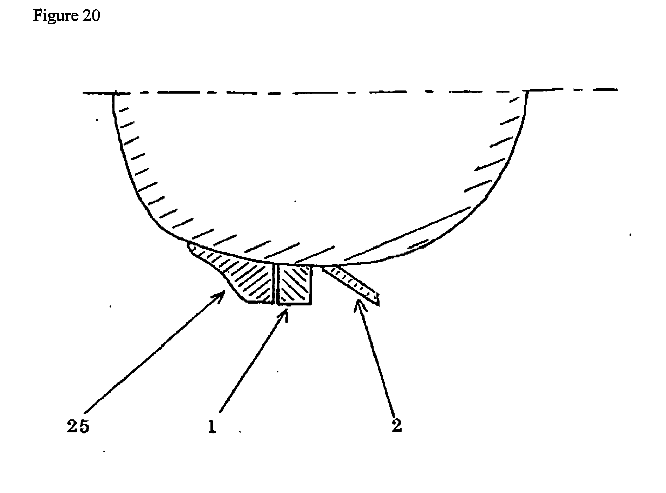

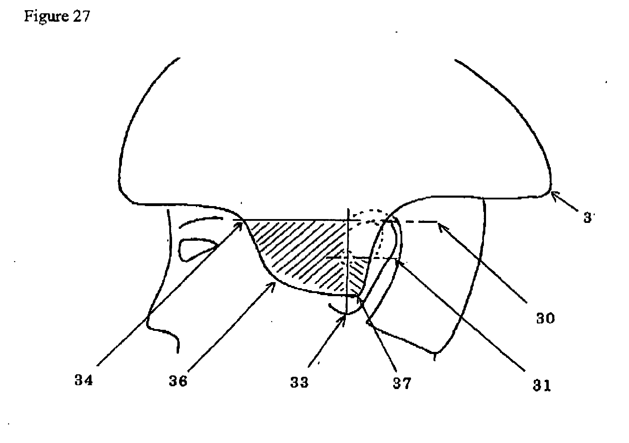

The following concerns the windshields B (25) which do not form part of the invention:
15. The above-described effect is achieved if the frond edgse of the windshields B (25) are located at least five centimeters forward of the centers of the ear holes (5). When the user is wearing a helmet (3), the wind flows rapidly above the centers of the user's ear holes in the vicinity of the earlobes. Accordingly, the windshields B (25) need not cover the windshields A (1) completely as seen from the front. Rather, all that is required is to locate the lower edges of the windshields B below the centers of the ear holes (5). The upper edges of the windshields are preferably in intimate contact with the lower edge of the helmet and when they are not in contact with the helmet (3), the upper edges should be located no more than 5 mm below the helmet's lower edge.
16. As shown in Embodiments 7 and 8 below, if the windshields are integrated with the helmet (3), the boundaries that define the windshields may become somewhat ambiguous. For the sake of convenience, it is assumed that one windshield comprises the portion forward of the center of the ear hole (5) below the top (30) of the base of the earlobe (4), and the portion rear of and below the center of the ear hole (5). The reason for distinguishing the two portions at the center of the ear hole (5) is that the rear portion is not necessary for noise reduction but may be required for ornamental purposes. See Figure 27 for the above.
17. As later shown in Embodiments 7 and 8, if each of the windshields A (1) and each of the windshields B (25) are integrated into a single element with the two no longer distinguishable from each other, Item 6 above is applied to the position of the rear end of the integral windshield A+B (35). However, if the rear end is positioned at the center of the ear hole or forward of the forwardmost part of the earlobe, the whistling sounds from winds will be reduced sufficiently. The provisions set forth in Items 15 and 16 are otherwise applied.
18. As shown in Figures 20, 21, and 22, the lateral thickness of the windshield B (26) should be tapered down to the front edge thereof. If the only intended use is to reduce the whistling sounds from winds when the user's head is tilted forward, this tapering is not necessary.
19. The line defined by the front edge of each windshield B (25) is preferably inclined rearward toward its bottom as seen in Figure 20. This effectively redirects the apparent headwind downward and slows the wind as it passes by the ear holes (5). Additionally, when the user's head is tilted forward as shown in Figure 24, that arrangement effectively prevents the wind passing below the lower end (26) of the helmet (3) from striking the front edge of the windshield B (25) and producing noise. As shown in Figures 27, 28, 29, and 30, it is possible to bend or curve this for aesthetic purposes. It should be noted that U.S. Patent No. 6,029,769 shows a "shaped fiber filter" having an identical inclination to that of the front edge of the windshield B (25). This, however, does not have the foregoing effect as the "outer abutments" prevents any wind from passing downward.
20. In the locations where the windshields A (1), the windshields B (25), the helmet (3), and the user's head either approach or come into contact with one another, it is more desirable to have as small a gap or step as possible existing therebetween. Particularly, it should be no longer than one centimeter long and 5 mm long or less, if possible.
21. It is preferable to support the windshields B (26) by connecting them to the lower end of the helmet (3). It is even more preferable to provide an attachment/detachment mechanism or a position adjustment mechanism. If thermoplastic elastomer is used for part of or the whole of the windshields, the user or the dealer can make fine adjustments of their shape to fit to the shape of the user's head. Thermoplastic elastomer may also be used for the windshields A (1) and the sound insulating plates (2).

### BRIEF DESCRIPTION OF THE ATTACHED DRAWINGS

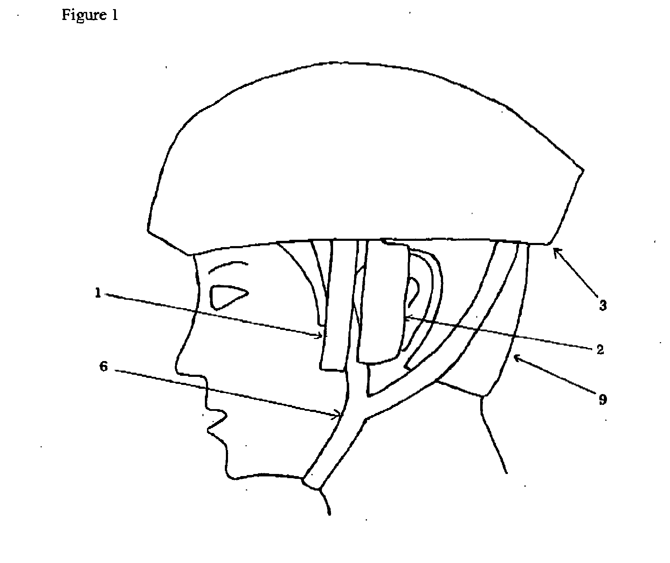

Figures 1, 2, and 3 show Embodiment 1.

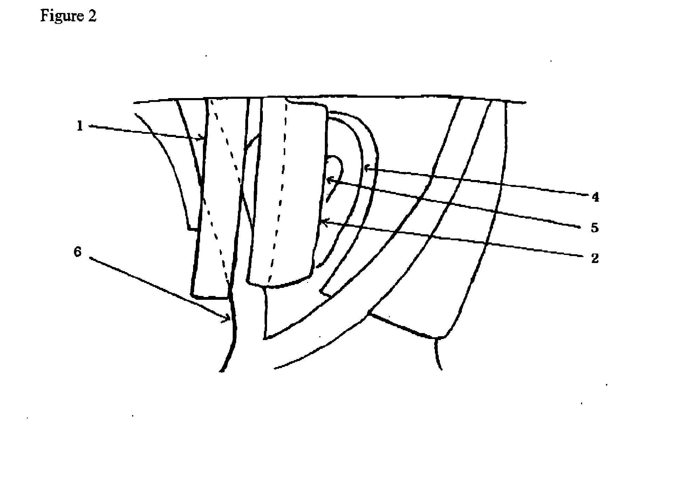

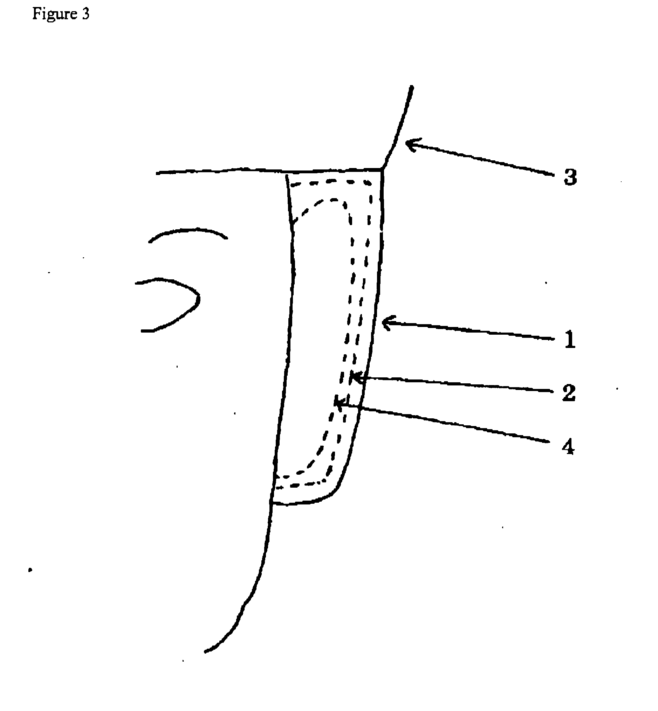

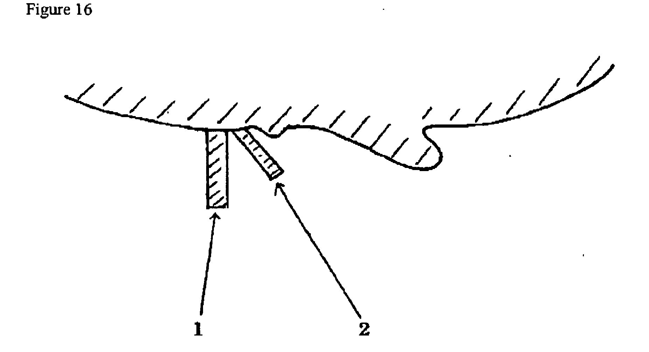

Figure 1 is a side view, showing the entire human head. Figure 2 is an enlarged side view of the vicinity of the apparatus. Figure 3 is an elevation view showing only the left part of the apparatus. The orientations of the windshield A (1) and the sound insulating plate (2) are as shown in Figure 16.

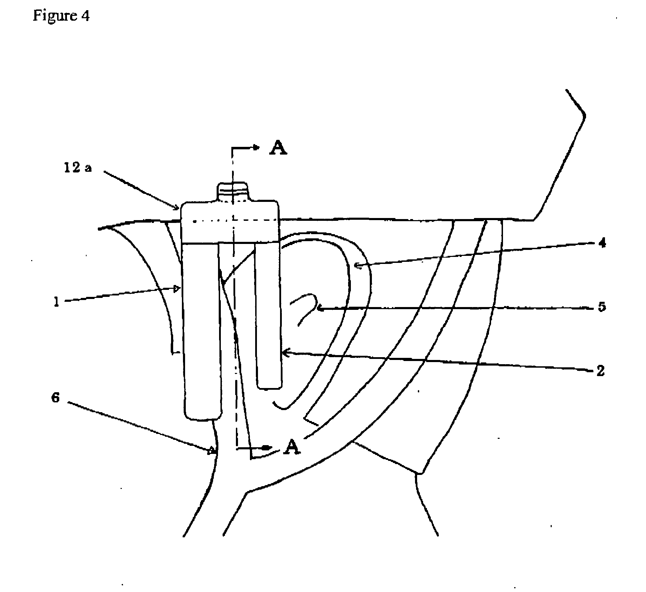

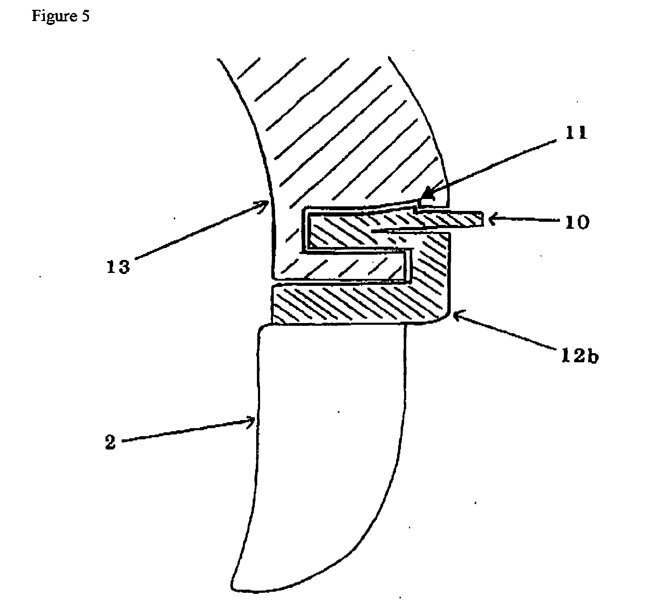

Figures 4 and 5 show Embodiment 2. Figure 4 is a side view. Figure 5 shows a cross-sectional view taken on line A-A of Figure 4. The user's head is not shown in the view. This shows the apparatus and its vicinity larger than the actual size. The gaps between the elements are shown larger than the actual sizes. The orientations of the windshield A (1) and the sound insulating plate (2) are as shown in Figure 15.

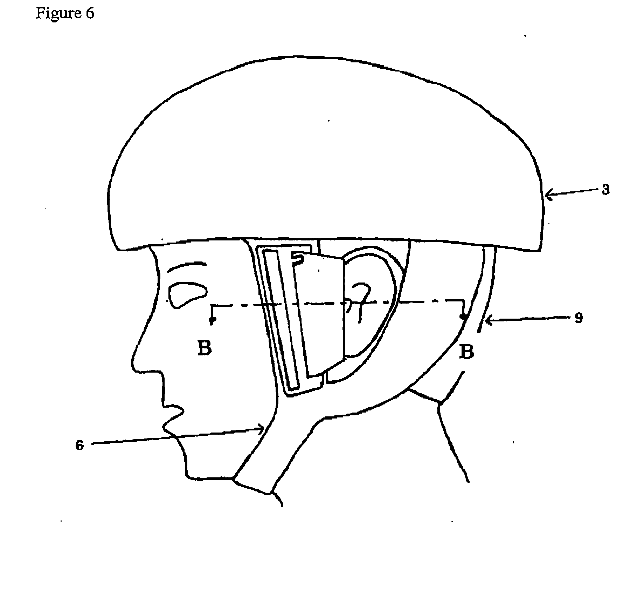

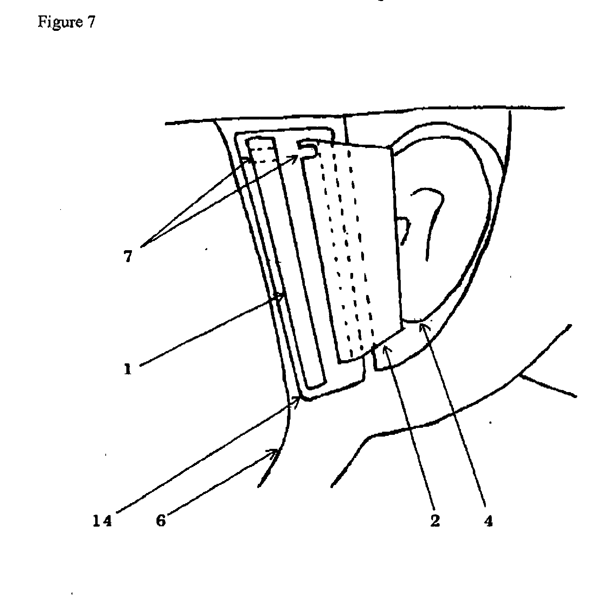

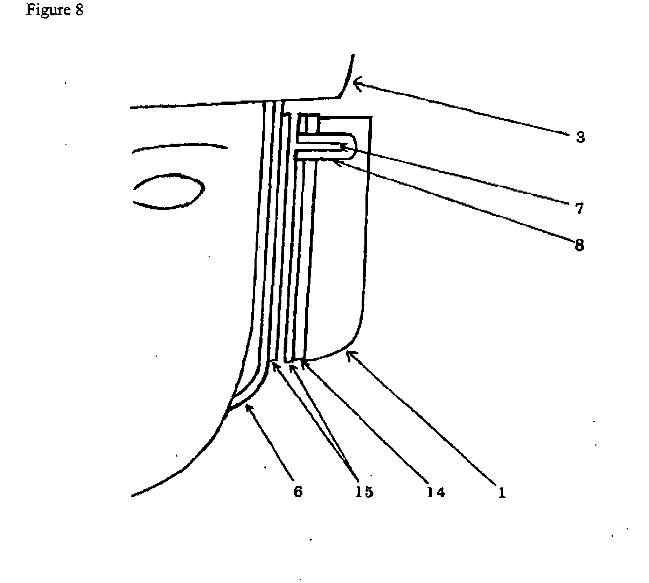

Figures 6, 7, and 8 show Embodiment 3. Figure 6 is a side view of Embodiment 3. This is an overall view. Figure 7 is an enlarged side view of the vicinity of the apparatus. Figure 8 is an elavation view of Embodiment 3. The chip strap (6) and the fabric hook-and-loop fastener (15) are shown thicker than their actual sizes. The side of the head and the chip strap (6) and the fabric hook-and-loop fastener (15) are in contact with each other and the two fabric hook-and-loop fastener (15) strips are in contact with each other in actuality. However, the view shows separations therebetween. Figure 16 shows a cross-sectional view taken on line B-B of Figure 6.

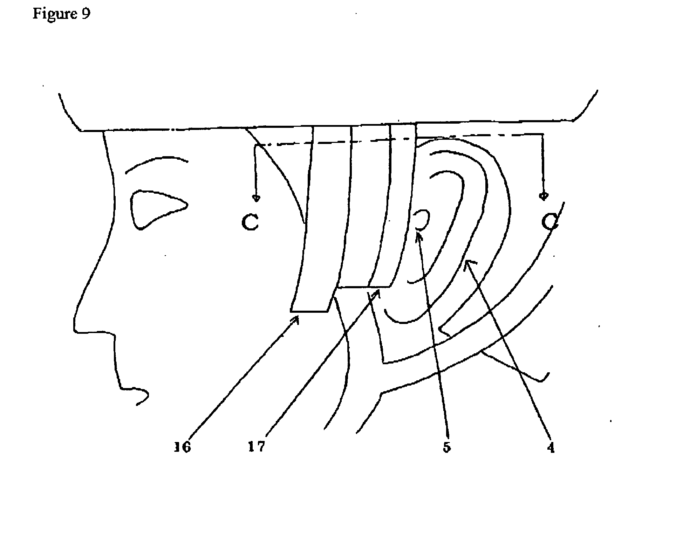

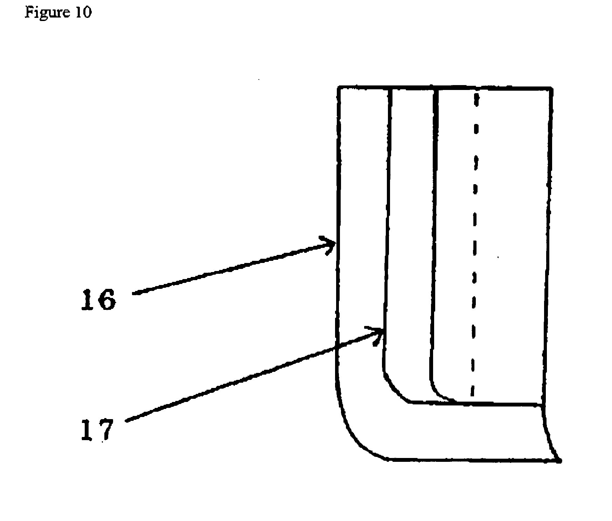

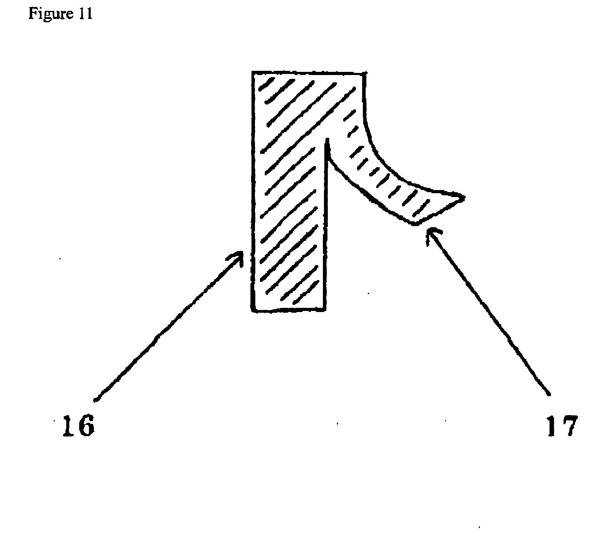

Figures 9, 10, and 11 show Embodiment 4. Figure 9 is a side view. This is an overall view. Figure 10 is a rear view. The helmet (3) and the user's head are omitted from the view. Figure 11 shows a cross-sectional view taken on line C-C of Figure 9. The user's head is omitted from the view.

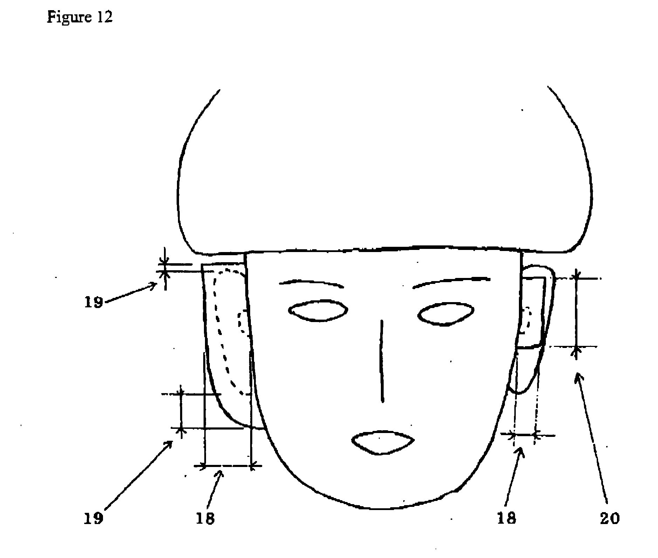

Figure 12 is an elevation view showing how to measure the dimensions of the windshields A (1). The windshield A (1) on the left in the view is of the maximum size and the windshield A (1) on the right in the view is of the minimum size.

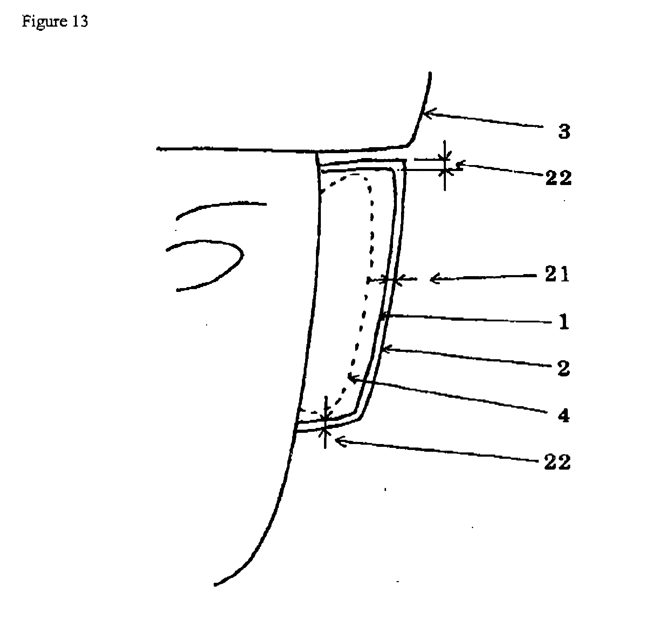

Figure 13 is an elevation view showing how to measure the amount by which the sound insulating plate 2 juts out beyond the windshield A (1).

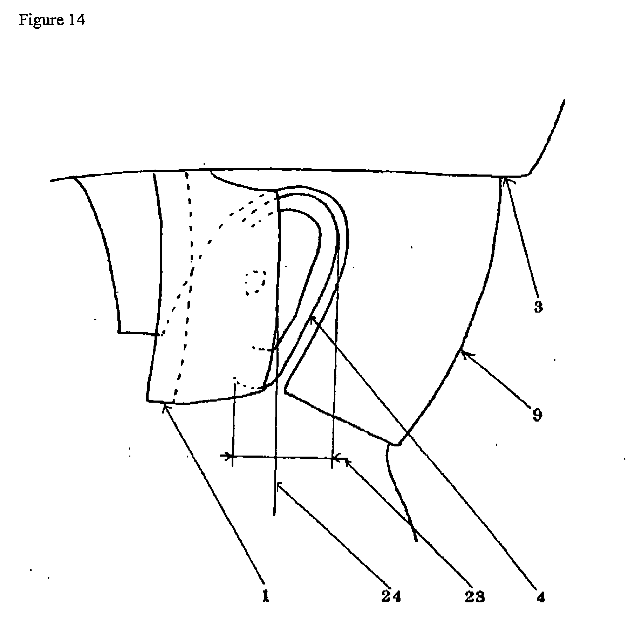

Figure 14 is a side view showing how to calculate the location of the rear end of the windshield A (1) and the front-to-rear length of the earlobe (4). For the sake of simplicity, the sound insulating plate (2) and the chin strap (6) are omitted from the view.
Figures 15, 16, 17, and 18 are cross-sectional views taken at the level of the ear hole (5), showing four exemplary orientations of the windshield A (1) and the sound insulating plate (2). For example, Figure 16 shows a cross-sectional view taken on line B-B of Figure 6.

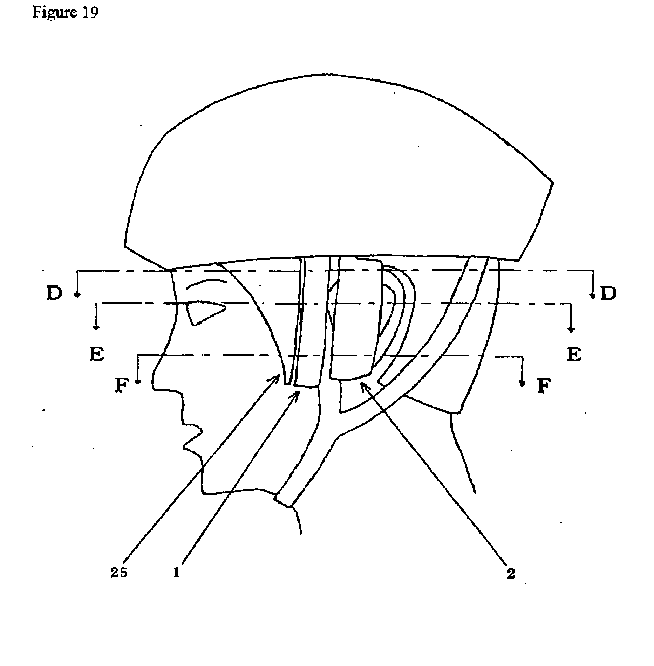

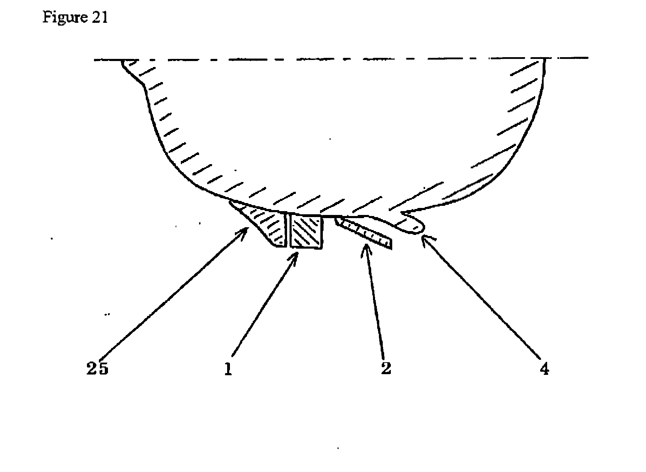

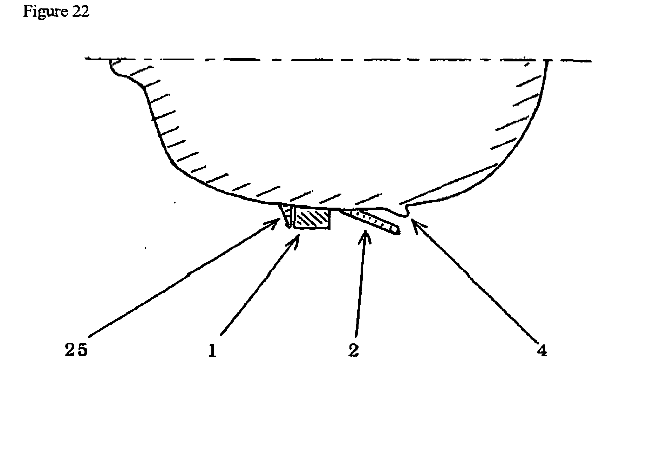

Figures 19 shows Embodiment 5. This is a side view. Figures 20, 21, and 22 show cross-sectional views taken of Figure 19. Figure 20 is taken on line D-D, Figure 21 on line E-E, and Figure 22 on line F-F. For the sake of simplicity, only the cross sections are shown.
Figure 23 shows the wind flow in the vicinity of the ears with the user's head tilted forward when only the windshields A (1) and the sound insulating plates (2) are provided and the windshields B (25) are omitted. Figure 24 shows the wind flow in the vicinity of the ears with the user's head tilted forward when the windshields B (25) are provided.

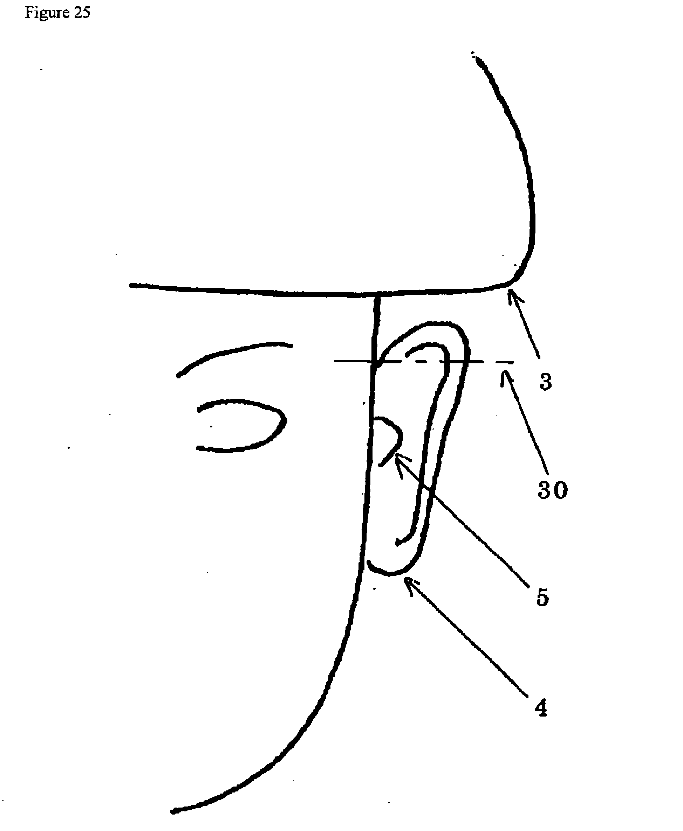

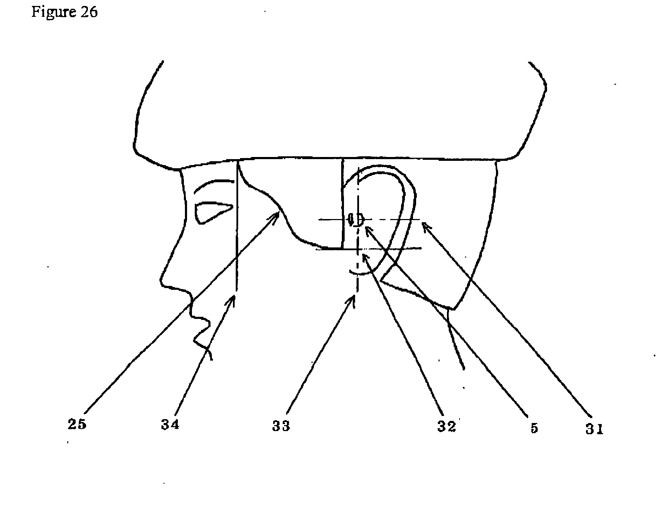

Figures 25, 26, and 27 show how various dimensions of the windshields B (25) are measured. Figure 25 is an elevation view showing the top of the base of the earlobe (4). Figure 26 shows a method of calculating the basic dimensions of the windshields B (25) and Figure 27 shows a method of defining the areas of the windshields B (25) when the boundaries are not clear.

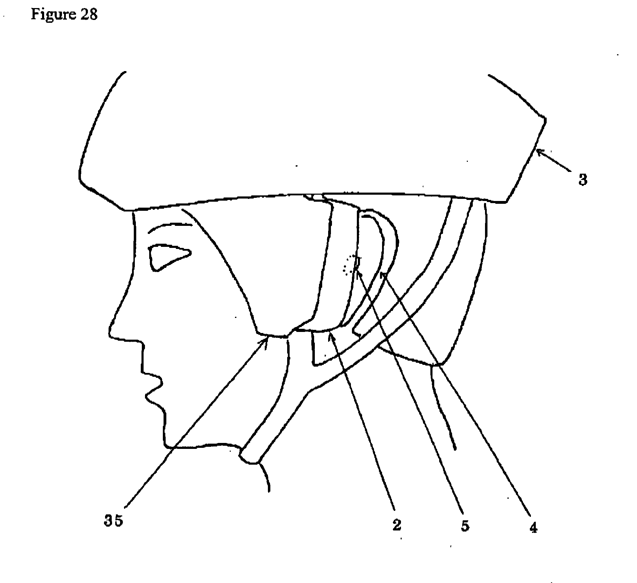

Figure 28 shows Embodiment 6.

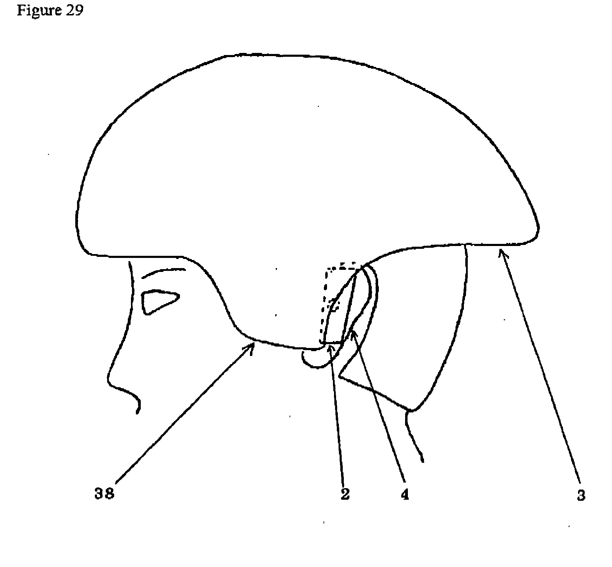

Figure 29 shows Embodiment 7.

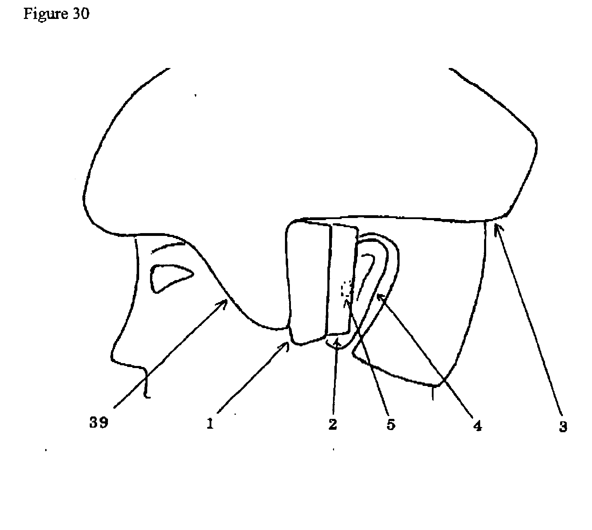

Figure 30 shows Embodiment 8.

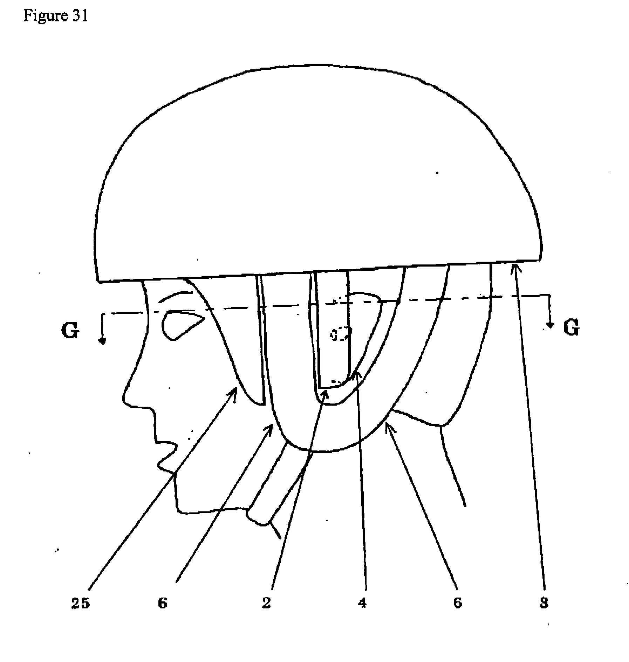

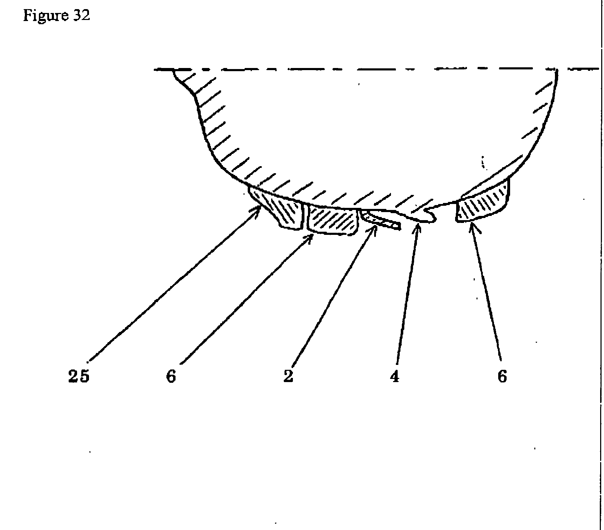

Figures 31 and 32 show Embodiment 9. Figure 31 is a side view, whereas Figure 32 is a cross-sectional view taken on line G-G of Figure 31, showing only the cross sections for the sake of simplicity.

### Legends:

- 1.: Windshield A
- 2.: Sound insulating plate
- 3.: Helmet
- 4.: User's earlobe
- 5.: User's ear hole
- 6.: Chin strap
- 7.: Hole to pass a temple of glasses through
- 8.: Sponge
- 9.: Hair
- 10.: Attachment/detachment lug
- 11.: Slip-off prevention mechanism
- 12a.: Mount A
- 12b.: Cross section of mount A
- 13.: Cross section of heimet
- 14.: Mount B
- 15.: Fabric hook-and-loop fastener tape
- 16.: Portion that functions as a windshield
- 17.: Portion that functions as a sound insulating plate
- 18.: Method of measuring dimensions, i.e., maximum and minimum lateral values, of windshield A (1)
- 19.: Method of measuring dimensions, i.e., maximum vertical values, of windshield A (1)
- 20.: Method of measuring dimensions, i.e., minimum vertical values, of windshield A (1)
- 21.: Method of measuring the amount by which sound insulating plate (2) juts out laterally beyond windshield A (1)
- 22.: Method of measuring the amount by which sound insulating plate (2) juts out vertically beyond windshield A (1)
- 23.: Method of measuring front-to-rear length of earlobe (4)
- 24.: Location of rear end of windshield A (1)
- 25.: Windshield B
- 26.: Lower end of helmet (3)
- 27.: User's temple region
- 28.: Front edge of windshield A (1)
- 29.: Wind path in the vicinity of ear in leaning forward position
- 30.: Vertical position of top of earlobe (4) base
- 31.: Vertical position of center of ear hole (3)
- 32.: Lower end of windshield B (25)
- 33.: Front-to-rear position of center of ear hole (5)
- 34.: Location of front end of windshield B (25)
- 35.: Windshield integrating windshield A (1) and windshield B (25)
- 36.: Section of what is considered windshield B (25) forward of center of ear hole (5)
- 37.: Section of what is considered windshield B (25) rear of center of ear hole (5)
- 38.: Portion integrated with helmet (3) that functions as windshield A (1) and windshield B (25)
- 39.: Portion integrated with helmet (3) that functions as windshield B (25)

### Embodiment 1

Windshields A (1) and sound insulating plates (2) are attached to the upper end of the dome of a helmet (3). This is suited for the type of helmets that cover the user's head above the ears and have a thin chinstrap. Bicycle helmets and some of motorcycle helmets fall into this category.

### Embodiment 2

This adds to the apparatus of Embodiment 1 a mechanism to detachably attach the windshields A (1) and the sound insulating plates (2). The windshields A (1) and the sound insulating plates (2) are connected to the helmet (3) via mounts A (12) and secured by slip-off prevention mechanisms. When the apparatus is not used, the attachment/detachment lugs (10) are pulled down to detach the apparatus.

### Embodiment 3

The windshields A (1) and the sound insulating plates (2) are attached to the chinstrap (6) of a helmet (3). This method is suited for a type of helmets that cover the user's head above the ears and have a wide, belt-type chinstrap. Horseracing helmets and some of motorcycle helmets fall into this category.

It is even more preferable for the apparatus to be made detachable. In the illustrated example, the windshields A (1) and the sound insulating plates (2) are attached to the mounts B (14), which are secured to the chinstrap (6) with fabric hook-and-loop fastener tapes (15). This renders the apparatus detachable.

### Embodiment 4

For those users who do not wear helmets, the apparatus may be secured with an arched support as in a headset, which does not form part of the invention.

### Embodiment 5

Figure 19 shows an embodiment where the windshields A (1), the sound insulating plates (2), and the windshields B (25) are attached to the helmet (3). The windshields B (25) are secured to the helmet (3).

### Embodiment 6

This integrates the windshield A (1) and the windshield B (25) of Embodiment 5.

### Embodiment 7

This integrates the windshields A (1) and the windshields B (25) with the helmet (3).

### Embodiment 8

This integrates the windshields B (25) with the helmet (3). As the windshields B reduce the whistling sounds from winds to some degree, one may usually use the helmet (3) without the windshields A (1) and the sound insulating plates (2), such that the windshields B may be attached only when it is necessary to particularly reduce the whistling sounds from winds.

### Embodiment 9

In this embodiment, the helmet (3) including a thick chinstrap is provided with the windshields B (25) and the sound insulating plates (2) only. The chinstrap (6) serves as the windshields A (1). This system is useful for helmets with chinstraps of thicknesses of approximately two centimeters or greater. Although the sound insulating plates (2) are provided in the illustrated embodiment, a significant reduction in the whistling sounds from winds may be obtained without these plates.

### INDUSTRIAL APPLICABILITY

The present invention effectively eliminates the whistling sounds from winds that would be otherwise heard by a person who is subjected to a steady apparent headwind. The present invention is applicable to all situations where this occurs.

Main intended users are riders of open-top vehicles. When riding an open-top vehicle, it is desirable to wear a helmet covering the entire head for safety. However, there still exist cases where helmets that expose ears are selected. Alternatively, there are cases where helmets are not worn. Accordingly, there is a need for the present invention.

In addition to the above, the present invention may be employed in working conditions of high winds or in sports where players move predominantly in forward directions.

## Claims

1. An apparatus provided on a helmet worn by a user for reducing the whistling sounds from winds comprising,
at least one windshield (1) for blocking forward of the user's ear an apparent headwind that would otherwise reach the ear hole (5), and
at least one sound insulating plate (2) for blocking noise generated by the vibration of the at least one windshield between the at least one windshield and the ear hole, **characterised in that** the at least one windshield and the at least one sound insulating plate are separated from each other in a front-to-rear direction.

2. An apparatus for reducing the whistling sounds from winds in accordance with claim 1, **characterized by** further comprising a mechanism for adjusting the positions in which the at least one windshield (1) and the at least one sound insulating plate (2) are attached according to the physical constitution of the user.

3. An apparatus for reducing the whistling sounds from winds in accordance with claim1, **characterized in that** the at least one windshield (1) is provided on a chinstrap (6) of the helmet.

4. An apparatus for reducing the whistling sounds from winds in accordance with claim 1, **characterized by** comprising two of the windshields (1) and two of the sound insulating plates (2).

## Patentansprüche

1. Vorrichtung, die an einem Helm angebracht ist, der von einem Benutzer getragen wird, um windbedingte Pfeifgeräusche zu vermindern, umfassend:
mindestens einen Windschild (1), um vor dem Ohr des Benutzers einen scheinbaren Gegenwind abzuwehren, der ansonsten die Ohröffnung (5) erreichen würde, und
mindestens eine schallisolierende Platte (2), um das Geräusch zu dämmen, das durch die Vibration des mindestens einen Windschildes (1) zwischen dem mindestens einen Windschild (1) und der Ohröffnung (5) erzeugt wird, zwischen dem mindestens einen Windschild (1) und der Ohröffnung (5),
**dadurch gekennzeichnet, dass** der mindestens eine Windschild (1) und die mindestens eine schallisolierende Platte (2) in einer Vor-Rück-Richtung voneinander getrennt sind.

2. Vorrichtung zur Verminderung windbedingter Pfeifgeräusche nach Anspruch 1, **dadurch gekennzeichnet, dass** sie ferner einen Mechanismus zum Einstellen der Positionen umfasst, in denen der mindestens eine Windschild (1) und die mindestens eine schallisolierende Platte (2) entsprechend der körperlichen Verfassung des Benutzers befestigt werden.

3. Vorrichtung zur Verminderung windbedingter Pfeifgeräusche nach Anspruch 1, **dadurch gekennzeichnet, dass** der mindestens eine Windschild (1) an einem Kinnband (6) des Helmes angebracht ist.

4. Vorrichtung zur Verminderung windbedingter Pfeifgeräusche nach Anspruch 1, **dadurch gekennzeichnet, dass** sie zwei Windschilde (1) und zwei schallisolierende Platten (2) umfasst.

## Revendications

1. Appareil monté sur un casque porté par un utilisateur pour réduire les bruits de sifflement du vent, comprenant :
au moins un écran de protection contre le vent (1) pour bloquer, à l'avant de l'oreille de l'utilisateur, un vent de face apparent qui atteindrait autrement l'orifice auriculaire (5), et
au moins une plaque isolante acoustique (2) pour bloquer le bruit généré par la vibration de l'au moins un écran de protection contre le vent (1) entre l'au moins un écran de protection contre le vent (1) et l'orifice auriculaire (5),
**caractérisé en ce que** l'au moins un écran de protection contre le vent (1) et l'au moins une plaque isolante acoustique (2) sont séparés l'un de l'autre dans une direction de l'avant vers l'arrière.

2. Appareil pour réduire les bruits de sifflement du vent selon la revendication 1, **caractérisé en ce qu'**il comprend en outre un mécanisme pour régler les positions auxquelles l'au moins un écran de protection contre le vent (1) et l'au moins une plaque isolante acoustique (2) sont fixés en fonction de la constitution physique de l'utilisateur.

3. Appareil pour réduire les bruits de sifflement du vent selon la revendication 1, **caractérisé en ce que** l'au moins un écran de protection contre le vent (1) est monté sur une jugulaire (6) du casque.

4. Appareil pour réduire les bruits de sifflement du vent selon la revendication 1, **caractérisé en ce qu'**il comprend deux écrans de protection contre le vent (1) et deux plaques isolantes acoustiques (2).
